# FASCICULE DE BREVET EUROPEEN

(11) **EP 3 706 656 B1**
(45) Date de publication et mention de la délivrance du brevet: **10.07.2024**
(21) Numéro de dépôt: 18819178.7
(22) Date de dépôt: 08.11.2018
(51) Int. Cl.: A61B 34/00, A61B 34/30, A61B 34/10, A61B 90/50, A61B 34/20

(54) **DISPOSITIF ROBOTISÉ POUR UNE INTERVENTION MÉDICALE MINI-INVASIVE SUR DES TISSUS MOUS**
ROBOTERVORRICHTUNG FÜR EINEN MINIMAL-INVASIVEN MEDIZINISCHEN EINGRIFF BEI WEICHTEILEN
ROBOTIC DEVICE FOR A MINIMALLY INVASIVE MEDICAL INTERVENTION ON SOFT TISSUES

(30) Priorité: 09.11.2017 FR 1760553
(43) Date de publication de la demande: 16.09.2020
(73) Titulaire: Quantum Surgical, 34000 Montpellier (FR)
(72) Inventeur: BLONDEL, Lucien, 34070 Montpellier (FR); BADANO, Fernand, 69006 Lyon (FR); NAHUM, Bertin, 34170 Castelnau-le-Lez (FR)
(74) Mandataire: Ipside
(86) Numéro de dépôt international: PCT/FR2018/052769
(87) Numéro de publication internationale: WO 2019/092372

(56) Documents cités:
- EP-A1- 2 468 207
- WO-A1-2016/127173
- WO-A1-2017/116512
- WO-A2-96/11624
- US-A1- 2008 186 378
- US-A1- 2010 063 514
- US-A1- 2011 257 514
- US-A1- 2015 366 546
- US-A1- 2017 265 947

## Description

### DOMAINE TECHNIQUE

La présente invention appartient au domaine des interventions médicales, et concerne plus particulièrement un dispositif robotisé pour les interventions médicales mini-invasives des tissus déformables d'un patient, par exemple pour le traitement ou le diagnostic sur des organes ou des structures anatomiques déformables.

### ÉTAT DE LA TECHNIQUE

Les interventions médicales (diagnostiques, thérapeutiques et/ou chirurgicales) par voie mini-invasive ou percutanée deviennent de plus en plus importantes, notamment en oncologie dans les traitements locaux du cancer, en agissant directement sur les cellules de l'organe atteint tel que le foie, le rein, le poumon, le pancréas, le sein, la prostate, etc.

En plus de l'oncologie, il existe de multiples gestes et applications médicales utilisant une voie d'abord mini-invasive ou percutanée, par exemple par l'insertion d'une aiguille : biopsies (prélèvement de tissus pour analyse pathologique), placement de drains (aspiration de fluides), injections de produits thérapeutiques (traitement de la douleur), etc.

Contrairement à la chirurgie ouverte ou conventionnelle qui peut nécessiter une incision de plusieurs dizaines de centimètres, les interventions médicales mini-invasives utilisent tout ou plus de petites incisions ou ouvertures au travers desquelles sont introduits un endoscope, une sonde, une aiguille ou d'autres instruments médicaux pour atteindre, visualiser et/ou traiter la zone anatomique ciblée.

Les interventions médicales mini-invasives peuvent apporter de nombreux bénéfices tels que la limitation de la douleur et du traumatisme opératoire, la diminution du saignement au cours d'une l'intervention chirurgicale, la réduction de la durée d'hospitalisation. Elles permettent de réaliser l'intervention médicale en chirurgie ambulatoire, ce qui permet une récupération plus rapide du patient, des cicatrices plus petites, une diminution du risque infectieux, etc.

En plus de la technique conventionnelle de résection chirurgicale avec des pinces, ciseaux et autres instruments médicaux, plusieurs technologies sont validées ou en cours d'évaluation pour la destruction de tissus par voie mini-invasive ou percutanée. On peut citer par exemple la chirurgie au laser, la cryothérapie, la radiofréquence, les micro-ondes, l'électroporation ou encore les ultrasons focalisés et la curiethérapie. La plupart de ces techniques ont en commun la réalisation d'une très petite incision et l'insertion jusqu'à la zone anatomique ciblée d'une ou plusieurs aiguilles, sondes ou électrodes pour délivrer un traitement de manière précise et localisée (traitement thermique, non-thermique ou radioactif).

Les interventions médicales effectuées par voie mini-invasive nécessitent le plus souvent l'insertion par l'opérateur d'un instrument médical à l'intérieur du corps du patient jusqu'à une certaine profondeur pour atteindre la zone anatomique ciblée. La réalisation de ces gestes est parfois longue et difficile car contrairement à la chirurgie ouverte, l'opérateur ne dispose pas toujours de la vision directe de l'anatomie du patient et de l'organe à traiter. Cela complique l'identification des structures anatomiques, le placement précis de l'instrument médical et l'évitement des structures anatomiques sensibles (nerfs, vaisseaux, organes sains, etc.).

Les chirurgiens peuvent utiliser des images médicales préopératoires (CT ou « Computerized Tomography », IRM ou « Imagerie par Résonance Magnétique », radiographie, etc.) réalisées à des fins de diagnostic pour faciliter le repérage de l'anatomie et la planification préalable de l'intervention médicale. Les images préopératoires fournissent une représentation de l'anatomie qui est valable à un instant donné, qui n'est cependant pas l'instant de réalisation de l'intervention médicale et est antérieur à celui-ci.

Pour introduire correctement l'instrument médical à l'intérieur du corps du patient jusqu'à la position et la profondeur voulues sans endommager les structures anatomiques sensibles pendant l'opération, l'opérateur doit pouvoir savoir où se trouve l'instrument médical à l'intérieur du corps du patient. Plusieurs systèmes et méthodes sont disponibles aujourd'hui pour déterminer la position et l'orientation de l'instrument médical pendant une intervention médicale mini-invasive lorsque la vision directe de l'anatomie n'est pas disponible au travers d'un microscope ou d'un endoscope.

Les systèmes de navigation (ou chirurgie assistée par ordinateur) guidés par l'image permettent de suivre en temps-réel la position et l'orientation d'un instrument médical en affichant un instrument médical virtuel superposé sur des images du corps du patient. Ils utilisent des technologies de localisation 3D pour repérer à la fois le patient et l'instrument médical, dont les plus répandues sont de type optique ou électromagnétique.

L'utilisation d'un système d'acquisition d'images est nécessaire avant, au début et/ou pendant l'intervention médicale pour acquérir une ou des images du patient (par scanner, IRM, radiographies, ultrasons, etc.). Avant de démarrer l'intervention médicale, ces images sont mises en correspondance avec la position réelle de l'anatomie du patient installé sur la table d'opération par différentes méthodes de recalage connues, telles que le recalage rigide ou déformable de points et/ou de surfaces remarquables, ou le référencement de la position du système d'acquisition d'images lui-même.

Les systèmes de navigation optique repèrent la position de l'instrument médical grâce à des caméras infrarouge et des émetteurs ou réflecteurs placés selon une géométrie connue sur l'instrument médical et sur le patient pour servir de référence et suivre ses mouvements.

Les systèmes de navigation électromagnétique repèrent la position de l'instrument médical grâce à un générateur de champ magnétique de faible intensité placé près du corps du patient, des capteurs qui peuvent être incorporés dans l'instrument médical et des capteurs de référence placés sur le patient. Ces systèmes de navigation électromagnétique sont compacts et ne souffrent pas du problème d'obstruction du champ de vue des systèmes de navigation optique. En revanche, ils nécessitent un environnement spécifique et contraignant lié à la présence d'un champ magnétique formé par le générateur de champ magnétique.

L'ensemble de ces systèmes de navigation connus, même s'ils peuvent améliorer la précision du geste médical par rapport à la méthode manuelle conventionnelle en fournissant en temps réel la position et l'orientation de l'instrument médical dans des images, présentent cependant des limitations fortes pour les interventions médicales mini-invasives sur des tissus déformables.

Une première limitation est que le geste final d'introduction de l'instrument médical jusqu'à la zone anatomique ciblée est réalisé manuellement par l'opérateur, ce qui rend le résultat dépendant de l'habileté de l'opérateur et ne permet pas d'atteindre une précision élevée.

Une seconde limitation est que le fonctionnement de ces systèmes de navigation suppose que les organes ou structures anatomiques ciblées ne se déplacent pas et ne se déforment pas entre le moment où l'examen de référence est réalisé et le moment où l'opérateur introduit l'instrument médical. Dans le cas où l'examen a été réalisé plusieurs jours avant l'intervention médicale et avec le patient dans une position différente sur la table d'opération de celle qu'il avait sur la table d'examen, les organes ou structures anatomiques ciblées peuvent avoir bougé ou s'être déformés et le décalage entre la position affichée et la position réelle de l'organe ou structure anatomique ciblée peut introduire une imprécision élevée. En outre, les organes ou structures anatomiques ciblées peuvent se déformer du simple fait de la respiration du patient, et les systèmes de navigation connus reposent sur le contrôle de la respiration par le patient, ce qui limite grandement la précision atteignable par ces systèmes de navigation.

Il existe également des dispositifs robotisés d'assistance au geste médical pour la chirurgie par voie mini-invasive.

Il est notamment connu du brevet US 8795188 un système pour une intervention médicale sur un patient qui comprend un robot, un dispositif d'enregistrement des mouvements du patient et une méthode pour prendre en compte de façon automatique des mouvements périodiques du patient, typiquement les mouvements de la cage thoracique dus à la respiration.

Toutefois, les variantes décrivant l'usage d'une technologie de navigation, ou d'un scan laser continu nécessitent une acquisition d'images avant l'opération et supposent que l'organe ou la structure anatomique ciblée ne se déplacent pas et ne se déforment pas par rapport à l'enveloppe externe du patient (peau). La variante décrivant l'usage d'images de type à rayons X pendant l'intervention nécessite la mise en place complexe et irradiante d'une acquisition d'images en continu.

En outre, les limitations décrites ci-avant en considérant le cas d'une intervention médicale nécessitant l'insertion d'un instrument médical à l'intérieur du corps du patient sont généralisables à des interventions médicales ne nécessitant pas d'introduire un instrument médical dans le corps du patient. Par exemple, dans le cas d'un instrument de traitement par ultrasons focalisés, il faut également pouvoir maîtriser le trajet des ondes ultrasonores à l'intérieur du corps du patient jusqu'à la zone anatomique ciblée à l'intérieur du corps dudit patient vers laquelle lesdites ondes ultrasonores doivent être focalisées.

La demande de brevet US 2015/366546 A1 décrit un bras robotisé à l'extrémité duquel est montée une sonde ultrasonore intégrant un guide pour une aiguille à biopsie. Le bras robotisé est destiné à être mis en oeuvre pour la réalisation de biopsies de la prostate. La sonde ultrasonore est conçue pour et destinée à être mise en contact intime avec le patient, ce qui confère un caractère intrusif à l'intervention médicale.

Les demandes de brevet EP 2468207 A1 et US 2011/0257514 A1 concernent des méthodes pour estimer le déplacement du cerveau après avoir réalisé une ouverture dans la boîte crânienne. Ces méthodes utilisent toutefois un système d'acquisition irradiant (tomodensitométrie, rayons X, IRM, tomographie par émission de positons, etc.) ou intrusif (sonde ultrasonore).

### EXPOSÉ DE L'INVENTION

La présente invention a pour objectif de remédier à tout ou partie des limitations des solutions de l'art antérieur, notamment celles exposées ci-avant, en proposant une solution qui permette d'assister l'opérateur à positionner un instrument médical par rapport à un organe ou une structure anatomique dans le corps d'un patient, dans le but d'effectuer un diagnostic ou traitement thérapeutique localisé, en prenant en compte le fait que l'organe ou la structure anatomique peut se déplacer ou se déformer à l'intérieur du corps du patient.

A cet effet, et selon un premier aspect, l'invention concerne un dispositif robotisé pour une intervention médicale sur un patient avec un instrument médical, comportant :
- un bras robot comportant plusieurs degrés de liberté, comportant une extrémité adaptée à recevoir l'instrument médical,
- un système d'acquisition d'images adapté à acquérir des informations de position de l'anatomie du patient,
- un support de mémorisation comportant un modèle biomécanique des structures anatomiques du corps humain,
- un circuit de traitement configuré pour transformer les paramètres d'une trajectoire à respecter par l'instrument médical pour réaliser l'intervention médicale dans le repère de référence associé au dispositif robotisé en fonction du modèle biomécanique et en fonction des informations de position de l'anatomie dudit patient, déterminer une consigne de position et une consigne d'orientation pour ledit instrument médical en fonction de la trajectoire,
- un circuit de contrôle configuré pour contrôler le bras robot pour placer ou assister dans le placement de l'instrument médical dans la consigne de position et la consigne d'orientation, caractérisé en ce que le système d'acquisition d'images est de type non irradiant et comporte au moins un équipement dit sans contact adapté à acquérir des informations de position sans contact avec le patient.

Grâce au bras robotisé, la précision et la répétabilité de positionnement de l'instrument médical sont largement supérieures à celle d'un opérateur. Ce gain en précision permet d'exécuter le traitement choisi par l'opérateur au plus près de l'organe ou de la structure anatomique ciblée, et donc d'en améliorer l'efficacité clinique. Il permet d'envisager de traiter des lésions encore inopérables car trop petites ou situées proches ou à l'intérieur de zones critiques. La précision et la répétabilité permettent aussi de réduire les risques de complications telles que des saignements, douleurs, et pertes de fonctions dues à l'endommagement de structures anatomiques sensibles présentes sur la trajectoire à respecter à la suite d'erreurs manuelles de positionnement de l'instrument médical.

Le dispositif robotisé utilise également la connaissance a priori d'un modèle biomécanique du corps humain.

Par « modèle biomécanique » du corps humain, on entend un modèle mathématique des différentes structures anatomiques (muscles, tendons, structures osseuses, organes, réseau vasculaire, etc.) du corps humain et donc du patient dans la zone anatomique considérée qui permet de modéliser les déformations desdites structures anatomiques ainsi que les interactions mécaniques entre lesdites structures anatomiques. Un tel modèle biomécanique permet donc, notamment, de déterminer les déformations et interactions mécaniques (et donc les déplacements) des structures anatomiques internes du patient induites, par exemple, par une modification de l'enveloppe externe dudit patient, une modification des positions des vaisseaux d'un organe, une modification de l'enveloppe externe d'un organe, etc. De telles modifications peuvent par exemple être induites par la respiration du patient (déplacement des organes induit par le mouvement de la cage thoracique et du diaphragme), par un changement de position dudit patient (déplacement des organes induit par gravité), par un contact avec un instrument médical (déformation locale), etc. La zone anatomique considérée correspond par exemple à la zone thoracique et/ou à la zone abdominale et/ou à la zone pelvienne du patient.

Ainsi, le dispositif robotisé utilise la trajectoire, le modèle biomécanique et les informations de position acquises pendant l'intervention médicale pour déterminer la position réelle d'une structure anatomique mobile et déformable dans le corps du patient, quels que soient la position du patient sur la table d'opération et le niveau de sa respiration. Cette fonctionnalité fiabilise grandement la réalisation de l'intervention médicale en évitant les erreurs dues à la compensation par l'opérateur des mouvements liés à la respiration et aux déformations internes des organes non prises en compte par les systèmes de navigation ou de robotique connus de l'art antérieur.

Pour toutes ces raisons, le dispositif robotisé est particulièrement adapté pour les interventions médicales mini-invasives sur des tissus déformables d'un patient.

Dans des modes particuliers de réalisation, le dispositif robotisé peut comporter en outre l'une ou plusieurs des caractéristiques suivantes, prises isolément ou selon toutes les combinaisons techniquement possibles.

Dans des modes particuliers de réalisation, le modèle biomécanique modélise les structures anatomiques du corps humain dans la zone thoracique et/ou la zone abdominale et/ou la zone pelvienne.

Selon l'invention, le système d'acquisition d'images est de type non irradiant.

En effet, grâce à la prise en compte du modèle biomécanique, le système d'acquisition d'images utilisé au cours de l'intervention peut être de type non irradiant. Par « non irradiant », on entend qu'aucun rayonnement ionisant (en particulier rayons X) n'est généré en direction du patient pour acquérir les images au cours de l'intervention médicale. La dose d'irradiation est donc fortement réduite, à la fois pour le patient et pour l'équipe médicale qui évolue à proximité du système d'acquisition d'images. En outre, le système d'acquisition d'images peut être beaucoup moins coûteux et encombrant que, par exemple un scanner CT, de sorte que le dispositif robotisé peut être utilisé y compris dans des salles opératoires de dimensions réduites et dépourvues de scanner CT, ce qui rend son utilisation beaucoup moins contraignante.

Selon l'invention, le système d'acquisition d'images comporte au moins un équipement dit sans contact adapté à acquérir des informations de position sans contact avec le patient.

Dans des modes particuliers de réalisation, le système d'acquisition d'images comporte l'un au moins des équipements sans contact suivants : une caméra stéréoscopique, une caméra à lumière structurée, une caméra à mesure de temps de vol, une caméra à mesure de profondeur, etc.

Dans des modes particuliers de réalisation, le système d'acquisition d'images est adapté à fournir des informations de position correspondant à la position d'une surface externe du corps du patient.

Selon l'invention, le système d'acquisition d'images est constitué par un ou des équipements sans contact, c'est-à-dire que le système d'acquisition d'images, et plus généralement le dispositif robotisé, comporte uniquement un ou plusieurs équipements sans contact et est dépourvu d'équipement avec contact pour l'acquisition d'images.

Dans des modes particuliers de réalisation, le circuit de contrôle est configuré pour contrôler le bras robot selon au moins un mode parmi les modes suivants : un mode automatique, un mode collaboratif, un mode suivi automatique, un mode suivi collaboratif, etc.

Dans des modes particuliers de réalisation, le circuit de traitement est configuré pour déterminer ou assister dans la détermination de la trajectoire de l'instrument médical en fonction d'images du patient.

Dans des modes particuliers de réalisation, le circuit de traitement est configuré pour régler ou assister dans le réglage de paramètres d'un traitement à effectuer au cours de l'intervention médicale par simulation des effets desdits paramètres en fonction d'images du patient.

Dans des modes particuliers de réalisation, le dispositif robotisé comporte un outil de guidage adapté à guider l'instrument médical, fixé ou destiné à être fixé à une extrémité du bras robot.

Dans des modes particuliers de réalisation, le dispositif robotisé comporte au moins un équipement d'interface homme-machine parmi les équipements suivants : un écran de visualisation, un écran de visualisation tactile, un clavier, des lunettes de vision 2D et/ou 3D, une manette de commande, un module de détection de mouvement, un module de commande vocale, etc.

Dans des modes particuliers de réalisation, le dispositif robotisé comporte au moins un équipement de repérage de point d'entrée parmi les équipements suivants : un instrument médical à pointe atraumatique, un module de visée laser, etc.

Dans des modes particuliers de réalisation, l'instrument médical est l'un des instruments médicaux suivants : une aiguille de biopsie, un cathéter, un endoscope, ou encore un instrument de traitement par ultrasons focalisés, par laser, par cryothérapie, par radiofréquence, par électroporation, par curiethérapie, etc.

Dans des modes particuliers de réalisation, le dispositif robotisé comporte un chariot mobile portant le bras robot, ledit chariot mobile comportant des moyens d'immobilisation.

### PRÉSENTATION DES FIGURES

L'invention sera mieux comprise à la lecture de la description suivante, donnée à titre d'exemple nullement limitatif, et faite en se référant aux figures qui représentent :
- Figure 1 : une représentation schématique d'un exemple de réalisation d'un dispositif robotisé pour les interventions médicales mini-invasives des tissus mous,
- Figure 2 : une représentation schématique d'une variante de réalisation du dispositif robotisé de la figure 1,
- Figure 3 : une représentation schématique d'un autre exemple de réalisation d'un dispositif robotisé.

Dans ces figures, des références identiques d'une figure à une autre désignent des éléments identiques ou analogues. Pour des raisons de clarté, les éléments représentés ne sont pas à l'échelle, sauf mention contraire.

### DESCRIPTION DÉTAILLÉE DE MODES DE RÉALISATION

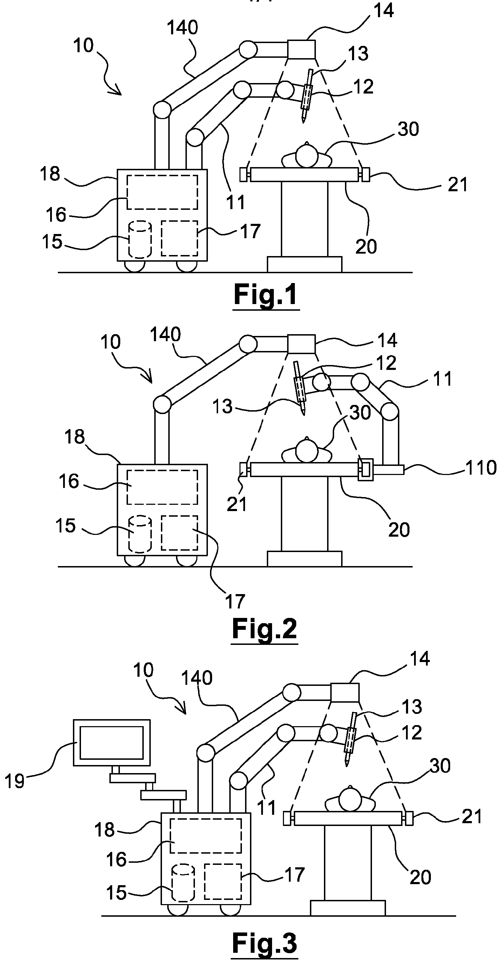

La figure 1 représente schématiquement un exemple de réalisation d'un dispositif robotisé 10 pour assister un opérateur lors d'une intervention médicale, par exemple de type mini-invasive sur des tissus mous.

Tel qu'illustré par la figure 1, le dispositif robotisé 10 comporte un bras robot 11 à plusieurs degrés de liberté. Le bras robot 11 comporte une extrémité adaptée à recevoir un instrument médical 13. Dans l'exemple illustré par la figure 1, l'instrument médical 13 est monté sur l'extrémité du bras robot 11 par l'intermédiaire d'un outil de guidage 12 adapté à guider ledit instrument médical 13. A cet effet, le bras robot 11 comporte au niveau de l'extrémité une interface adaptée à recevoir ledit outil de guidage 12.

De préférence, le bras robot 11 comporte au moins 6 degrés de liberté afin de permettre d'avoir de grandes plages de contrôle, dans l'espace, de la position et de l'orientation de l'outil de guidage 12 par rapport à un patient 30, par exemple allongé sur une table d'opération 20.

L'outil de guidage 12 est adapté à guider l'instrument médical 13, c'est-à-dire à contraindre le déplacement dudit instrument médical 13 par rapport audit outil de guidage 12. Par exemple, l'outil de guidage 12 est un coulisseau adapté à guider l'instrument médical 13 en translation pour contraindre le déplacement dudit instrument médical 13 par exemple lors de son insertion dans le corps du patient 30.

L'outil de guidage 12 est par exemple fixé de manière amovible sur le bras robot 11, lequel est de préférence adapté à recevoir différents types d'outils de guidage 12, par exemple associés à des instruments médicaux 13 différents et/ou à des gestes médicaux différents.

L'interface du bras robot 11 peut comporter, par exemple, un mécanisme détrompeur pour assurer un montage correct des outils de guidage 12 sur le bras robot 11. Dans des modes préférés de réalisation, l'interface peut comporter en outre un système électronique permettant de reconnaître automatiquement l'outil de guidage 12 monté par l'opérateur pour ensuite utiliser dans les calculs les caractéristiques de l'outil de guidage 12 telles que sa référence, ses dimensions, son poids, son centre de masse, et toute autre donnée utile pour sa fonction ou sa performance d'utilisation.

Le dispositif robotisé 10 est de préférence adapté à recevoir, sur un outil de guidage 12 porté par le bras robot 11, tout type d'instrument médical 13, en particulier tout type d'instrument médical mis en oeuvre pour les interventions médicales mini-invasives sur des tissus mous. Par exemple, Le dispositif robotisé 10 est de préférence adapté à recevoir et déplacer l'un au moins des instruments médicaux chirurgicaux suivants :
- une aiguille de biopsie,
- un cathéter,
- un endoscope,
- un instrument de traitement par ultrasons focalisés,
- un instrument de traitement par laser,
- un instrument de traitement par cryothérapie,
- un instrument de traitement par radiofréquence,
- un instrument de traitement par électroporation,
- un instrument de traitement par curiethérapie, etc.

Le dispositif robotisé 10 comporte également un circuit de contrôle 16 adapté à contrôler le bras robot 11 pour modifier la position et l'orientation de l'outil de guidage 12 dans un repère de référence associé au dispositif robotisé 10. Le circuit de contrôle 16 comporte par exemple un ou plusieurs processeurs et des moyens de mémorisation (disque dur magnétique, mémoire électronique, disque optique, etc.) dans lesquels est mémorisé un produit programme d'ordinateur, sous la forme d'un ensemble d'instructions de code de programme à exécuter pour contrôler le bras robot 11. Alternativement ou en complément, le circuit de contrôle 16 comporte un ou des circuits logiques programmables (FPGA, PLD, etc.), et/ou un ou des circuits intégrés spécialisés (ASIC, etc.), et/ou un ensemble de composants électroniques discrets, etc., adaptés à contrôler ledit bras robot 11.

Grâce au circuit de contrôle 16, au bras robot 11 et à l'outil de guidage 12 porté par le bras robot 11, l'instrument médical 13 peut être positionné, orienté et guidé avec une précision beaucoup plus importante que dans le cas d'un instrument médical 13 manipulé directement par un opérateur.

Dans l'exemple illustré par la figure 1, le dispositif robotisé 10 comporte un chariot 18 mobile, par exemple monté sur roues, sur lequel est monté le bras robot 11. De telles dispositions sont particulièrement avantageuses en ce qu'il est alors particulièrement aisé de déplacer le bras robot 11 d'un côté à l'autre de la table d'opération, d'une salle opératoire à une autre, etc. Le chariot 18 comporte des moyens d'immobilisation (non représentés sur les figures) permettant d'immobiliser le chariot 18 par rapport à la table d'opération 20. Les moyens d'immobilisation peuvent être de tout type adapté, et peuvent comporter notamment des freins sur les roues, des patins ou pieds rétractables, des systèmes d'attache mécanique à la table d'opération 20, des systèmes d'attache mécanique au sol, etc.

Rien n'exclut cependant, suivant d'autres exemples, d'avoir le bras robot 11 monté directement sur la table d'opération, de manière amovible ou permanente (auquel cas la table d'opération fait partie intégrante du dispositif robotisé 10). La figure 2 représente schématiquement une variante de réalisation du dispositif robotisé 10 dans laquelle le bras robot 11 est monté de manière amovible sur la table d'opération 20. Dans l'exemple illustré par la figure 2, le bras robot 11 est monté sur un support 110 réalisant une liaison mécanique rigide avec des rails 21 de la table d'opération 20.

Tel qu'illustré par les figures 1 et 2, le dispositif robotisé 10 comporte également un système 14 d'acquisition d'images adapté à acquérir des informations de position de l'anatomie du patient 30 dans le repère de référence associé au dispositif robotisé 10, ou dans un repère différent dudit repère de référence pour lequel la matrice de passage vers ledit repère de référence est connue a priori ou peut être déterminée. Dans des modes préférés de réalisation, le système 14 d'acquisition d'images est de type non irradiant, afin de limiter la dose d'irradiation subie par le patient 30 et par l'équipe médicale.

Le système 14 d'acquisition d'images permet d'acquérir des informations de position de l'anatomie du patient 30. Les informations de position de l'anatomie du patient 30 correspondent par exemple à la position de la surface externe du corps du patient 30 dans le repère de référence, la position de la structure osseuse dudit corps du patient 30 dans le repère de référence, la position d'un organe ou de vaisseaux à l'intérieur dudit corps du patient 30 dans le repère de référence, etc.

Le système 14 d'acquisition d'images comporte un ou plusieurs équipements dits sans contact adaptés à acquérir des informations de position sans contact avec le patient 30.

Dans des modes particuliers de réalisation, le système 14 d'acquisition d'images comporte l'un au moins des équipements sans contact suivants :
- une caméra stéréoscopique,
- une caméra à lumière structurée,
- une caméra à mesure de temps de vol (« Time of Flight caméra » ou « ToF caméra » dans la littérature anglo-saxonne),
- une caméra à mesure de profondeur (par exemple une caméra RGB-D), etc.

De tels équipements sans contact permettent par exemple d'acquérir des informations de position représentatives de la position de la surface externe du corps du patient 30 par rapport à l'équipement sans contact.

Dans des modes particuliers de réalisation non-revendiqués, le système 14 d'acquisition d'images comporte l'un au moins des équipements avec contact suivants :
- une sonde ultrasonore (acquisition avec contact non intrusive),
- un endoscope (acquisition avec contact intrusive), etc.

De tels équipements avec contact permettent par exemple d'acquérir des informations de position représentatives de la position d'un organe ou de vaisseaux à l'intérieur du corps du patient 30.

Le système 14 d'acquisition d'images est par exemple intégré au bras robot 11 ou monté à l'extrémité dudit bras robot 11.

Dans les exemples illustrés par les figures 1 et 2, le système 14 d'acquisition d'images est monté sur un support distinct du bras robot 11. Le support est par exemple un bras articulé 140, éventuellement motorisé auquel cas il forme un bras robot distinct du bras robot 11 portant l'outil de guidage 12 de l'instrument médical 13. Dans l'exemple illustré par la figure 1, le bras articulé 140 est porté, comme le bras robot 11, par le chariot 18 mobile. Dans l'exemple illustré par la figure 2, le bras articulé 140 portant le système 14 d'acquisition d'images est porté par un chariot 18 mobile.

Rien n'exclut en outre, suivant d'autres exemples, d'avoir un système 14 d'acquisition d'images porté par l'opérateur pour acquérir des informations de position de l'anatomie du patient 30.

La position et l'orientation dans l'espace du système 14 d'acquisition d'images sont par exemple connues dans le repère de référence du dispositif robotisé 10, soit par la connaissance de sa géométrie lorsqu'il est porté par le bras robot 11, soit par l'utilisation d'un système de localisation 3D tel qu'un navigateur optique, électromagnétique ou autre.

Tel qu'illustré par les figures 1 et 2, le dispositif robotisé 10 comporte également un support de mémorisation 15 mémorisant un modèle biomécanique des structures anatomiques du corps humain. Dans les exemples illustrés par les figures 1 et 2, le support de mémorisation 15 est représenté comme distinct du circuit de contrôle 16. Le support de mémorisation 15 peut cependant également être, suivant d'autres exemples de réalisation, l'un des moyens de mémorisation dudit circuit de contrôle 16.

Il est à noter que le modèle biomécanique du corps humain n'est pas nécessairement spécifique au patient 30 considéré, et peut être un modèle biomécanique d'un patient générique, par exemple de même sexe, taille, corpulence, etc. que le patient 30 considéré sur lequel doit être réalisé le geste médical. Le modèle biomécanique inclut de préférence les principales structures anatomiques de la zone thoraco-abdomino-pelvienne telles que les parois thoracique et abdominale, les muscles, les tendons, les os et articulations, les organes, le réseau vasculaire, etc., ainsi que leurs modèles de déformation et leurs interactions mécaniques. Le modèle biomécanique prend en compte également, de préférence, les effets de la gravité en fonction de la position du patient 30.

De tels modèles biomécaniques sont connus dans la littérature scientifique, voir par exemple les publications suivantes :
- « SOFA : A Multi-Model Framework for Interactive Physical Simulation », F. Faure et al., Soft Tissue Biomechanical Modeling for Computer Assisted Surgery - Studies in Mechanobiology, Tissue Engineering and Biomaterials, Volume 11, Springer,
- « A Personalized Biomechanical Model for Respiratory Motion Prédiction », B. Fuerst et al., International Conférence on Medical Image Computing and Computer Assisted Intervention, 2012,
- « Patient-Specific Biomechanical Model as Whole-Body CT Image Registration Tool », Mao Li et al., Medical Image Analysis, 2015, May, pages 22-34.

Le modèle biomécanique peut par exemple être créé par la transcription d'une base de données d'images médicales en trois dimensions (scans CT, IRM, etc.). La géométrie des structures d'intérêt peut être extraite des images médicales par des algorithmes de segmentation et de reconstruction. L'analyse de la base de données d'images permet de calculer une géométrie moyenne des composants du modèle biomécanique ainsi que les principaux paramètres de déformation représentatifs de l'ensemble des images médicales de la base de données. Il est possible d'assigner à chacune des structures des caractéristiques mécaniques et conditions aux limites différentes pour en créer un modèle biomécanique. Le modèle biomécanique inclut préférentiellement une modélisation du système musculo-squelettique composé des os, des muscles, des tendons, des ligaments et des cartilages.

Tel qu'illustré par les figures 1 et 2, le dispositif robotisé 10 comporte également un circuit de traitement 17. Le circuit de traitement 17 est configuré pour déterminer, en fonction du modèle biomécanique des structures anatomiques du corps humain et en fonction des informations de position acquises par le système 14 d'acquisition d'images, une consigne de position et une consigne d'orientation pour l'outil de guidage 12.

Le circuit de traitement 17 comporte par exemple un ou plusieurs processeurs et des moyens de mémorisation (disque dur magnétique, mémoire électronique, disque optique, etc.) dans lesquels est mémorisé un produit programme d'ordinateur, sous la forme d'un ensemble d'instructions de code de programme à exécuter pour déterminer la consigne de position et la consigne d'orientation. Alternativement ou en complément, le circuit de traitement 17 comporte un ou des circuits logiques programmables (FPGA, PLD, etc.), et/ou un ou des circuits intégrés spécialisés (ASIC, etc.), et/ou un ensemble de composants électroniques discrets, etc., adaptés à déterminer la consigne de position et la consigne d'orientation.

Dans les exemples illustrés par les figures 1 et 2, le circuit de traitement 17 est représenté comme distinct du circuit de contrôle 16. Le circuit de traitement 17 peut cependant, suivant d'autres exemples de réalisation, être confondu avec ou utiliser des équipements également utilisés par ledit circuit de contrôle 16. En outre, le support de mémorisation 15 est représenté comme distinct du circuit de traitement 17. Le support de mémorisation 15 peut cependant également être, suivant d'autres exemples de réalisation, l'un des moyens de mémorisation dudit circuit de traitement 17.

La consigne de position et la consigne d'orientation pour l'outil de guidage 12 sont déterminées en outre en fonction d'une trajectoire à respecter par l'instrument médical 13 lors de l'intervention médicale.

Dans le cas d'une intervention médicale nécessitant d'introduire l'instrument médical 13 dans le corps du patient 30, la trajectoire correspond au trajet qui doit être parcouru par l'instrument médical 13 à l'intérieur du corps du patient 30, selon laquelle ledit instrument médical doit être guidé lors de l'intervention médicale. La trajectoire correspond par exemple à la position d'un point d'entrée, par exemple sur la surface externe de l'anatomie du patient 30, par lequel l'instrument médical 13 doit pénétrer dans le corps du patient 30, ainsi que la position d'un point cible à l'intérieur du patient 30, au niveau de la structure anatomique ciblée, à atteindre avec ledit instrument médical 13. Le point d'entrée et le point cible sont par exemple mémorisés sous la forme de coordonnées dans un repère associé à l'anatomie du patient 30.

Dans le cas d'une intervention médicale ne nécessitant pas d'introduire l'instrument médical 13 dans le corps du patient 30, par exemple dans le cas d'un instrument de traitement par ultrasons focalisés, la trajectoire correspond au trajet qui doit être parcouru par les ondes ultrasonores à l'intérieur du corps du patient 30. La trajectoire correspond par exemple à la position d'un point d'entrée, par exemple sur la surface externe de l'anatomie du patient 30, par lequel les ondes ultrasonores doivent pénétrer dans le corps du patient 30, ainsi que la position d'un point cible à l'intérieur du patient 30 vers lequel les ondes ultrasonores doivent être focalisées. Le point d'entrée et le point cible sont par exemple mémorisés sous la forme de coordonnées dans un repère associé à l'anatomie du patient 30.

La trajectoire peut être prédéterminée par d'autres moyens que le dispositif robotisé 10, auquel cas elle est par exemple mémorisée dans le support de mémorisation 15 avant l'intervention médicale. Alternativement ou en complément, la trajectoire peut également être déterminée au moyen du dispositif robotisé 10, tel que décrit dans la suite de la description.

Le circuit de traitement 17 intègre des algorithmes de mise en correspondance du modèle biomécanique avec les informations de position de l'anatomie du patient 30 fournies par le système 14 d'acquisition d'images. Ainsi, le circuit de traitement 17 peut déterminer la position et l'orientation du patient 30 dans le repère de référence associé au dispositif robotisé 10. Le circuit de traitement 17 peut également déterminer la position du point d'entrée et la position du point cible de la trajectoire dans ledit repère de référence en tenant compte des déformations des structures anatomiques (par gravité terrestre, respiration, contact mécanique avec un instrument médical, etc.) du patient 30 par rapport aux structures anatomiques du patient 30 considérées pour déterminer ladite trajectoire. Par exemple, un algorithme permet de propager les mouvements de la surface de la peau au volume interne et de calculer correctement la position des structures anatomiques internes. Suivant un autre exemple, il est possible de déterminer la position et la déformation d'un organe à partir d'informations de position des vaisseaux de cet organe. Suivant un autre exemple, il est possible de déterminer la position et la déformation d'un organe à partir d'informations de position de la surface externe dudit organe. L'acquisition des informations de position de l'anatomie du patient 30 et le calcul de mise en correspondance du modèle biomécanique avec lesdites informations de position de l'anatomie du patient 30 sont de préférence effectués en temps-réel ou quasi temps-réel, de sorte que la position du point d'entrée et la position du point cible dans le repère de référence peuvent être actualisées en temps-réel ou quasi temps-réel pour suivre les mouvements et déformations des structures anatomiques du patient 30. Cette actualisation peut également être effectuée au cours de l'insertion de l'instrument médical 13 dans le corps du patient 30, afin de tenir compte des déformations induites par le déplacement dudit instrument médical 13.

Après avoir déterminé les paramètres de la trajectoire (positions du point d'entrée et du point cible) dans le repère de référence associé au dispositif robotisé 10, ou simultanément à cette détermination, le circuit de traitement 17 détermine une consigne de position et une consigne d'orientation de l'outil de guidage 12 permettant de respecter ladite trajectoire.

Le circuit de contrôle 16 peut alors contrôler le bras robot 11 pour placer ou assister l'opérateur dans le placement l'outil de guidage 12 dans ladite consigne de position et ladite consigne d'orientation déterminées par le circuit de traitement 17. Dans des modes préférés de réalisation, le circuit de contrôle 16 est adapté à contrôler le bras robot 11 selon au moins un mode parmi les modes suivants :
- un mode automatique,
- un mode collaboratif,
- un mode suivi automatique,
- un mode suivi collaboratif.

En mode automatique, le circuit de contrôle 16 déplace le bras robot 11 de sa position et orientation courante vers la consigne de position et la consigne d'orientation en calculant automatiquement la trajectoire entre la position courante et la consigne de position.

En mode collaboratif, le circuit de contrôle 16 déplace le bras robot 11 dans la direction des efforts exercés par l'opérateur, ces efforts pouvant être exercés sur l'outil de guidage 12 ou sur un des axes du bras robot 11. Les efforts sont mesurés et calculés grâce à un ou plusieurs capteurs (non représentés sur les figures) équipant l'extrémité du bras robot 11 et/ou chacun de ses axes. Des contraintes géométriques peuvent être intégrées au mode collaboratif pour restreindre les mouvements du bras robot 11 afin de faciliter le geste médical. Par exemple, les mouvements peuvent être contraints dans une zone, à l'extérieur d'une zone, le long d'un axe ou d'une courbe, autour d'un point, etc. La contrainte peut être définie par tout type de forme géométrique et de comportement associé (inclusion/exclusion). En mode collaboratif, le circuit de contrôle 16 assiste l'opérateur dans le placement de l'outil de guidage 12 dans la consigne de position et la consigne d'orientation.

En mode suivi (ou « tracking » dans la littérature anglo-saxonne), le circuit de contrôle 16 déplace le bras robot 11 dans la direction des mouvements du patient 30 pour placer l'outil de guidage 12 dans la consigne de position et la consigne d'orientation actualisées en temps-réel ou quasi temps-réel par le circuit de traitement 17. Dans ce cas, l'instrument médical 13 bouge dans le repère de référence associé au dispositif robotisé 10, mais reste sensiblement immobile au cours du temps dans un repère lié à l'organe ciblé.

En mode suivi collaboratif, le circuit de contrôle 16 déplace le bras robot 11 dans la direction des mouvements du patient 30 avec une flexibilité autour de la position commandée. L'opérateur peut exercer par exemple des efforts sur l'outil de guidage 12 et dévier légèrement et temporairement la position de l'outil de guidage 12 par rapport à la consigne de position et à la consigne d'orientation. Le bras robot 11 exerce des efforts opposés à ceux de l'opérateur qui visent à ramener l'outil de guidage 12 dans la consigne de position et la consigne d'orientation dès lors qu'aucun effort n'est exercé par l'opérateur. Le niveau de flexibilité peut par exemple être ajustable par un paramètre de raideur ou de distance.

La figure 3 représente schématiquement un mode préféré de réalisation, dans lequel le dispositif robotisé 10 comporte un équipement d'interface 19 homme-machine. Dans l'exemple illustré par la figure 3, l'équipement d'interface 19 homme-machine est un écran de visualisation, de préférence tactile. L'équipement d'interface 19 homme-machine permet à l'opérateur de contrôler le dispositif robotisé 10 et, éventuellement, de visualiser des images en relation avec le geste médical à réaliser. Par exemple, l'équipement d'interface 19 homme-machine peut être utilisé lors de la planification du geste médical, afin d'établir la trajectoire de l'instrument médical 13, ou encore pour visualiser la progression de l'instrument médical 13 dans le corps du patient 30, en affichant par exemple la position en temps-réel ou quasi temps-réel de l'instrument médical 13 par rapport à la position du point cible dans le repère de référence. L'équipement d'interface 19 homme-machine peut également être utilisé pour afficher les images fournies par le système 14 d'acquisition d'images.

Dans l'exemple illustré par la figure 3, l'équipement d'interface 19 homme-machine est porté par un chariot 18 mobile portant également le bras robot 11. Rien n'exclut, suivant d'autres exemples, d'avoir l'équipement d'interface 19 homme-machine porté par une console séparée, ou monté sur un rail de la table d'opération 20, par exemple de manière amovible. En outre, d'autres équipements d'interface 19 homme-machine peuvent être considérés, alternativement ou en complément. Par exemple, l'équipement d'interface 19 homme-machine peut comporter une souris, un clavier, un pavé tactile, une manette de commande (« joystick »), un module de détection de mouvement sans contact qui repère les mouvements de la main, des doigts, de la tête ou des yeux de l'opérateur, ou encore un module de commande vocale, etc. En outre, des lunettes de vision 2D et/ou 3D peuvent également remplacer ou compléter l'écran de visualisation.

Afin de sécuriser l'utilisation du dispositif robotisé 10, l'équipement d'interface 19 homme-machine peut également comporter un module d'acquittement (pédale avec ou sans fil, boîtier à bouton, télécommande, interrupteur sur le bras robot 11) pour sécuriser les déplacements du bras robot 11, qui sont alors conditionnés à l'activation dudit module d'acquittement.

Dans des modes préférés de réalisation, le circuit de traitement 17 est configuré pour déterminer ou assister l'opérateur dans la détermination de la trajectoire de l'instrument médical 13 en fonction d'images du patient. Dans la suite de la description, on décrit un exemple non limitatif de mise en oeuvre du dispositif robotisé 10 pour planifier une intervention médicale nécessitant d'introduire l'instrument médical dans le corps du patient 30.

L'intervention médicale peut être définie par exemple par la trajectoire, l'instrument médical 13 à utiliser et les paramètres de traitement. La trajectoire est par exemple constituée d'un point cible situé dans l'organe à traiter et d'un point d'entrée situé par exemple au niveau de la peau. L'instrument médical 13 est défini par plusieurs propriétés telles que sa longueur, son diamètre, sa forme géométrique 3D, etc. Les paramètres de traitement peuvent inclure des réglages d'une technologie d'ablation comme la puissance du courant délivré, le temps de traitement, le diamètre de la zone, les marges en distance, etc.

Le dispositif robotisé 10 peut par exemple charger des images du patient 30 (scanner CT, PET ou « Positron Emission Tomography », IRM, radiographie, ultrasons, etc.) à partir du réseau de l'hôpital ou d'un réseau externe (informatique en nuage), ou à partir d'un support de mémorisation externe (clé USB, CD, DVD, etc.) et permettre de les visualiser par exemple sur l'équipement d'interface 19 homme-machine selon des plans de coupes en deux dimensions et par des images reconstruites en trois dimensions. Par exemple, des algorithmes de recalage rigide et non-rigide permettent de fusionner plusieurs images d'un même patient 30 afin de fournir à l'opérateur toutes les informations anatomiques et fonctionnelles nécessaires à la planification de l'intervention médicale. L'opérateur peut alors planifier une ou plusieurs trajectoires selon l'intervention médicale à réaliser. Dans le cas d'une ablation par électroporation irréversible par exemple, le dispositif robotisé 10 permet de créer des trajectoires exactement parallèles pour optimiser l'efficacité du traitement.

Les positions du point cible et du point d'entrée de la trajectoire sont par exemple identifiées manuellement dans les images par l'opérateur. La trajectoire de l'instrument médical 13 dans l'anatomie peut être visualisée dans les images et modifiée pour assurer que l'extrémité de l'instrument médical 13 atteigne un point cible optimal et que l'insertion de l'instrument médical 13 n'endommage pas des structures anatomiques sensibles entre le point d'entrée et le point cible. Pour faciliter le processus décisionnel de planification, le dispositif robotisé 10 peut intégrer des algorithmes de segmentation qui identifient automatiquement les contours et volumes de certains organes d'intérêt, des nerfs, des artères, veines et vaisseaux, des os, ainsi que des lésions à traiter. Alternativement, le dispositif robotisé 10 peut déterminer automatiquement le point cible et le point d'entrée. Par exemple, le point cible est calculé par des méthodes de reconnaissance de formes et à partir des paramètres du traitement et du volume de la lésion ciblée. Le point d'entrée peut être calculé par des méthodes d'optimisation de critères tels que la distance entre la trajectoire de l'instrument médical 13 et des structures anatomiques sensibles et la position par rapport à des zones préférentielles d'insertion définies au niveau de la peau. Alternativement ou en complément, le dispositif robotisé 10 peut également accumuler au fur et à mesure de son utilisation de grandes quantités de données de planification qu'il réutilise et analyse pour proposer par un algorithme d'intelligence artificielle une sélection de points d'entrée et de points cibles optimaux.

Dans des modes préférés de réalisation, le circuit de traitement 17 est configuré pour régler ou assister l'opérateur dans le réglage des paramètres du traitement à effectuer au cours de l'intervention médicale, par simulation des effets desdits paramètres en fonction d'images du patient. Par exemple, le dispositif robotisé 10 peut, à partir des paramètres du traitement, des informations de trajectoires et des instruments médicaux 13, calculer les effets du traitement sur l'anatomie et permettre d'en visualiser une simulation précise sur les images du patient 30. Pour une ablation thermique par exemple, le calcul peut prendre en compte notamment la présence de vaisseaux adjacents et leur impact refroidissant (« heat-sink effect »). L'opérateur peut alors ajuster les données de planification pour optimiser le résultat clinique du traitement.

Le processus de planification décrit ci-dessus permet donc de planifier une grande variété de gestes médicaux tels que la chirurgie au laser, l'ablation par cryothérapie, radiofréquence, micro-ondes ou électroporation, la curiethérapie, l'endoscopie, et toute technique nécessitant l'insertion d'un ou plusieurs instruments médicaux 13 dans le corps d'un patient 30. Le processus de planification décrit ci-dessus permet également de planifier une grande variété de gestes médicaux ne nécessitant pas d'introduire un instrument médical 13 dans le corps d'un patient 30, comme par exemple dans le cas d'un traitement par ultrasons focalisés.

L'ensemble des données nécessaires à la planification peuvent être sauvegardées par le dispositif robotisé 10 dans des moyens de mémorisation du circuit de traitement 17 ou dans le support de mémorisation 15, ou sur un support de mémorisation externe et rechargées ultérieurement soit pour en modifier des éléments soit pour exécuter le traitement le jour de l'opération avec le dispositif robotisé 10.

On décrit à présent un exemple de mise en oeuvre du dispositif robotisé 10 pour réaliser un geste médical préalablement planifié, nécessitant d'insérer un instrument médical 13 dans le corps du patient 30.

Avant de démarrer l'opération, le dispositif robotisé 10 est amené dans la salle et placé à côté du patient 30. Dans le cas d'un bras robot 11 monté sur un chariot 18 mobile, le dispositif robotisé 10 est immobilisé préalablement à une phase de repérage du patient 30.

L'opérateur pilote ensuite le dispositif robotisé 10, par exemple par l'intermédiaire de l'équipement d'interface 19 homme-machine, pour lancer la phase de repérage du patient 30. La phase de repérage du patient 30 vise à déterminer la position du patient 30 dans le repère de référence associé au dispositif robotisé 10, mais également la position du point d'entrée et du point cible, ainsi que la consigne de position et la consigne d'orientation de l'outil de guidage 12, en utilisant le modèle biomécanique, les informations de position fournies par le système 14 d'acquisition d'images et la trajectoire planifiée.

Une fois la position du patient 30 connue et mise en correspondance avec les images préopératoires, l'opérateur lance la phase de positionnement, qui vise à placer l'outil de guidage 12 dans la consigne de position et la consigne d'orientation adaptées pour l'intervention médicale à réaliser.

Par exemple, le dispositif robotisé 10 prend en compte les dimensions de l'outil de guidage 12, la position du point d'entrée, et la direction vers le point cible pour positionner automatiquement le bras robot 11 de telle sorte que l'outil de guidage soit aligné sur la trajectoire choisie, à une distance de sécurité au point d'entrée ajustable. L'opérateur peut alors piloter le bras robot 11, par exemple en mode collaboratif pour ajuster la position de l'outil de guidage 12 au plus près du point d'entrée tout en maintenant l'alignement sur la trajectoire, puis bloquer les mouvements du bras robot 11 et enfin insérer un instrument médical 13 au travers de l'outil de guidage 12.

Dans des modes préférés de réalisation, le dispositif robotisé 10 comporte un équipement de repérage (non représenté sur les figures). Ainsi, grâce au dispositif robotisé 10, l'opérateur peut repérer précisément le point d'entrée sur la peau du patient 30, au niveau duquel une incision doit être réalisée. Par exemple, l'équipement de repérage de point d'entrée correspond à un instrument médical à pointe atraumatique qui est inséré dans l'outil de guidage 12, ou à un module de visée laser intégré dans un instrument médical 13 ou dans l'outil de guidage 12.

Après avoir effectué l'incision, l'opérateur peut débuter la phase de guidage en insérant l'instrument médical 13 au travers de l'outil de guidage 12 jusqu'à ce que l'extrémité de l'instrument médical atteigne le point cible planifié. La gestion de la profondeur d'insertion peut être basée simplement sur la longueur de l'instrument médical 13 et/ou sur un système de butée mécanique intégré à l'outil de guidage 12. Alternativement ou en complément, l'outil de guidage 12 peut comporter un capteur permettant de renseigner la profondeur d'insertion de l'instrument médical 13. Le dispositif robotisé 10 peut alors afficher en temps-réel ou quasi temps-réel la position de l'instrument médical 13 dans les images et fournir des messages à l'opérateur lorsque le point cible est proche, atteint ou dépassé. Dans une autre variante, l'instrument médical 13 est attaché mécaniquement à l'outil de guidage 12 et le bras robot 11 insère automatiquement l'instrument médical 13 jusqu'au point cible planifié.

Au cours de l'insertion, le circuit de traitement 17 peut utiliser le modèle biomécanique du patient 30 pour estimer les déformations locales des organes ou structures anatomiques traversées par l'instrument médical 13 et les prendre en compte pour actualiser la position du point cible.

En fonction des besoins de l'opération pendant la phase de guidage, le dispositif robotisé 10 est par exemple activé en mode suivi ou en mode suivi collaboratif pour maintenir la position de l'outil de guidage 12 par rapport à l'anatomie visée quels que soient les mouvements du patient 30. Le dispositif robotisé 10 peut également être activé en mode collaboratif pendant la phase de guidage, en appliquant ou non des contraintes géométriques. Le mode collaboratif contraint sur l'axe de la trajectoire est par exemple utile à la réalisation de biopsies étagées.

Lorsque l'instrument médical 13 a atteint le point cible, l'intervention médicale planifiée peut être exécutée à des fins de diagnostic ou de traitement localisé : par exemple le prélèvement d'un tissu pour une biopsie, l'acheminement d'azote liquide pour une cryothérapie, la génération d'un courant électrique pour une ablation par radiofréquence, l'injection de sources radioactives pour une curiethérapie, la visualisation directe de l'anatomie et l'insertion d'instruments médicaux dans le canal de travail de l'endoscope pour une chirurgie endoscopique, etc.

À tout moment pendant ou après la phase de guidage, l'opérateur peut vérifier l'exécution correcte de l'insertion de l'instrument médical 13 par une imagerie de contrôle. En fonction de l'équipement disponible à l'hôpital et dans la salle d'opération, un examen de la zone anatomique d'intérêt peut être effectué avec un appareil d'imagerie fixe ou mobile (scanner CT, IRM, arceau de radiologie, sonde ultrasonore, etc.). Dans des modes préférés de réalisation, les images sont directement transférées au dispositif robotisé 10, dont le circuit de traitement 17 comporte par exemple des algorithmes de recalage qui permettent de fusionner automatiquement ces images intra-opératoires avec les images préopératoires. Le dispositif robotisé 10 affiche alors les informations de planification superposées aux images intra-opératoires pour évaluer l'avancement ou l'efficacité du traitement et déterminer les corrections à apporter si nécessaire.

Le circuit de traitement 17 peut également comporter, dans des modes préférés de réalisation, des algorithmes de segmentation pour identifier automatiquement une zone nécrosée, la comparer avec la zone planifiée, calculer et afficher les marges obtenues en diamètre ou en volume, et indiquer le diamètre ou volume restant à traiter. Le dispositif robotisé 10 peut également, de manière optionnelle, proposer les informations nécessaires au traitement complémentaire, telles que les positions et paramètres d'une ou plusieurs trajectoires d'ablation additionnelles.

De manière plus générale, il est à noter que les modes de mise en oeuvre et de réalisation considérés ci-dessus ont été décrits à titre d'exemples non limitatifs, et que d'autres variantes sont par conséquent envisageables dans le cadre du champ de protection de la revendication 1.

Notamment, l'invention a été décrite en considérant que l'instrument médical 13 est monté sur le bras robot 11 par l'intermédiaire d'un outil de guidage 12. Il est cependant à noter que le dispositif robotisé 10 peut également être mis en oeuvre sans utiliser d'outil de guidage 12. Par exemple, l'utilisation d'un outil de guidage 12 n'est pas nécessaire dans le cas où l'instrument médical 13 ne doit pas être introduit dans le corps du patient 30, par exemple dans le cas d'un traitement externe par ultrasons focalisés. En outre, dans le cas où l'instrument médical 13 doit être inséré dans le corps du patient 30, l'utilisation d'un outil de guidage 12 est requise surtout si c'est l'opérateur qui insère l'instrument médical 13 dans le corps du patient 30, mais pas forcément si c'est le bras robot 11 qui insère automatiquement l'instrument médical 13 dans le corps du patient 30.

## Revendications

1. - Dispositif robotisé (10) pour une intervention médicale sur une zone anatomique située à l'intérieur du corps d'un patient (30) avec un instrument médical (13), comportant :
- un bras robot (11) comportant plusieurs degrés de liberté, comportant une extrémité adaptée à recevoir l'instrument médical,
- un système (14) d'acquisition d'images adapté à acquérir des informations de position de l'anatomie du patient,
- un support de mémorisation (15) comportant un modèle biomécanique des structures anatomiques du corps humain,
- un circuit de traitement (17) configuré pour
transformer les paramètres d'une trajectoire à respecter par l'instrument médical pour réaliser l'intervention médicale dans le repère de référence associé au dispositif robotisé en fonction du modèle biomécanique et en fonction des informations de position de l'anatomie dudit patient,
déterminer une consigne de position et une consigne d'orientation pour ledit instrument médical en fonction de la trajectoire,
- un circuit de contrôle (16) configuré pour contrôler le bras robot (11) pour placer ou assister dans le placement de l'instrument médical dans la consigne de position et la consigne d'orientation,
**caractérisé en ce que** le système (14) d'acquisition d'images est de type non irradiant et comporte au moins un équipement dit sans contact adapté à acquérir des informations de position sans contact avec le patient (30).

2. - Dispositif robotisé (10) selon la revendication 1, dans lequel le modèle biomécanique modélise les structures anatomiques du corps humain dans la zone thoracique et/ou la zone abdominale et/ou la zone pelvienne.

3. - Dispositif robotisé (10) selon l'une des revendications 1 à 2, dans lequel le système (14) d'acquisition d'images comporte l'un au moins des équipements sans contact suivants :
- une caméra stéréoscopique,
- une caméra à lumière structurée,
- une caméra à mesure de temps de vol,
- une caméra à mesure de profondeur.

4. - Dispositif robotisé (10) selon l'une des revendications 1 à 3, dans lequel le système (14) d'acquisition d'images est adapté à fournir des informations de position correspondant à la position d'une surface externe du corps du patient (30).

5. - Dispositif robotisé (10) selon l'une des revendications 1 à 4, dans lequel le circuit de contrôle (16) est configuré pour contrôler le bras robot selon au moins un mode parmi les modes suivants :
- un mode automatique,
- un mode collaboratif,
- un mode suivi automatique,
- un mode suivi collaboratif.

6. - Dispositif robotisé (10) selon l'une des revendications 1 à 5, dans lequel le circuit de traitement (17) est configuré pour déterminer ou assister dans la détermination de la trajectoire de l'instrument médical en fonction d'images du patient.

7. - Dispositif robotisé (10) selon l'une des revendications 1 à 6, dans lequel le circuit de traitement (17) est configuré pour régler ou assister dans le réglage de paramètres d'un traitement à effectuer au cours de l'intervention médicale par simulation des effets desdits paramètres en fonction d'images du patient.

8. - Dispositif robotisé (10) selon l'une des revendications 1 à 7, comportant un outil de guidage (12) adapté à guider l'instrument médical (13), fixé ou destiné à être fixé à une extrémité du bras robot (11).

9. - Dispositif robotisé (10) selon la revendication 8, dans lequel le circuit de contrôle (16) est configuré pour contrôler le bras robot (11) selon un mode suivi collaboratif de sorte que, lorsqu'un opérateur effectue des efforts sur le bras robot (11) pour dévier l'outil de guidage (12) par rapport à la consigne de position et la consigne d'orientation, le bras robot (11) exerce des efforts opposés à ceux effectués par l'opérateur, de sorte à ramener l'outil de guidage (12) dans la consigne de position et la consigne d'orientation dès lors qu'aucun effort n'est exercé par ledit opérateur.

10. - Dispositif robotisé (10) selon l'une des revendications 8 à 9, dans lequel l'outil de guidage (12) comprend un capteur de mesure d'une profondeur d'insertion de l'instrument médical (13).

11. - Dispositif robotisé (10) selon l'une des revendications 1 à 10, comportant au moins un équipement d'interface (19) homme-machine parmi les équipements suivants :
- un écran de visualisation,
- un écran de visualisation tactile,
- un clavier,
- des lunettes de vision 2D et/ou 3D,
- une manette de commande,
- un module de détection de mouvement,
- un module de commande vocale.

12. - Dispositif robotisé (10) selon l'une des revendications 1 à 11, comportant au moins un équipement de repérage de point d'entrée parmi les équipements suivants :
- un instrument médical à pointe atraumatique,
- un module de visée laser.

13. - Dispositif robotisé (10) selon l'une des revendications 1 à 12, dans lequel l'instrument médical (13) est l'un des instruments médicaux suivants :
- une aiguille de biopsie,
- un cathéter,
- un endoscope,
- un instrument de traitement par ultrasons focalisés,
- un instrument de traitement par laser,
- un instrument de traitement par cryothérapie,
- un instrument de traitement par radiofréquence,
- un instrument de traitement par électroporation,
- un instrument de traitement par curiethérapie.

14. - Dispositif robotisé (10) selon l'une des revendications 1 à 13, comportant un chariot (18) mobile portant le bras robot (11), ledit chariot mobile comportant des moyens d'immobilisation.

15. - Dispositif robotisé (10) selon l'une des revendications 1 à 14, dans lequel le circuit de traitement (17) est configuré pour utiliser le modèle biomécanique pour estimer des déformations locales des structures anatomiques traversées par l'instrument médical (13) et pour actualiser la position d'un point cible de la trajectoire que doit suivre l'instrument médical (13) en fonction des déformations locales estimées.

## Patentansprüche

1. Robotisierte Vorrichtung (10) für einen medizinischen Eingriff an einem anatomischen Bereich, der sich im Inneren des Körpers eines Patienten (30) befindet, mit einem medizinischen Instrument (13), umfassend:
- einen Roboterarm (11), der mehrere Freiheitsgrade umfasst und ein Ende umfasst, das dazu ausgelegt ist, das medizinische Instrument aufzunehmen,
- ein Bilderfassungssystem (14), das dazu ausgelegt ist, Positionsinformationen über die Anatomie des Patienten zu erfassen,
- ein Speichermedium (15), das ein biomechanisches Modell der anatomischen Strukturen des menschlichen Körpers umfasst,
- eine Verarbeitungsschaltung (17), die dazu konfiguriert ist,
die Parameter einer vom medizinischen Instrument einzuhaltenden Bewegungsbahn umzuwandeln, um den medizinischen Eingriff in dem der Robotervorrichtung zugeordneten Referenz-Koordinatensystem in Abhängigkeit von dem biomechanischen Modell und in Abhängigkeit von den Positionsinformationen über die Anatomie des Patienten durchzuführen,
einen Positionssollwert und einen Ausrichtungssollwert für das medizinische Instrument in Abhängigkeit von der Bewegungsbahn zu bestimmen,
- eine Steuerschaltung (16), die dazu konfiguriert ist, den Roboterarm (11) zu steuern, um das medizinische Instrument in den Positionssollwert und den Ausrichtungssollwert zu platzieren oder beim Platzieren davon zu helfen,
**dadurch gekennzeichnet, dass** das Bilderfassungssystem (14) vom strahlungsfreien Typ ist und mindestens ein berührungsloses Gerät umfasst, das dazu ausgelegt ist, Positionsinformationen zu erfassen, ohne den Patienten (30) dabei zu berühren.

2. Robotisierte Vorrichtung (10) nach Anspruch 1, wobei das biomechanische Modell die anatomischen Strukturen des menschlichen Körpers im Brustbereich und/oder im Bauchbereich und/oder im Beckenbereich modelliert.

3. Robotisierte Vorrichtung (10) nach einem der Ansprüche 1 bis 2, wobei das Bilderfassungssystem (14) mindestens eines der folgenden berührungslosen Geräte umfasst:
- eine stereoskopische Kamera,
- eine Strukturlichtkamera,
- eine Time-of-Flight-Kamera,
- eine tiefenmessende Kamera.

4. Robotisierte Vorrichtung (10) nach einem der Ansprüche 1 bis 3, wobei das Bilderfassungssystem (14) dazu ausgelegt ist, Positionsinformationen zu liefern, die der Position einer Außenfläche des Körpers des Patienten (30) entsprechen.

5. Robotisierte Vorrichtung (10) nach einem der Ansprüche 1 bis 4, wobei die Steuerschaltung (16) dazu konfiguriert ist, den Roboterarm gemäß mindestens einem Modus aus den folgenden Modi zu steuern:
- einem automatischen Modus,
- einem kollaborativen Modus,
- einem automatischen Verfolgungsmodus,
- einem kollaborativen Verfolgungsmodus.

6. Robotisierte Vorrichtung (10) nach einem der Ansprüche 1 bis 5, wobei die Verarbeitungsschaltung (17) dazu konfiguriert ist, die Bewegungsbahn des medizinischen Instruments in Abhängigkeit von Bildern des Patienten zu bestimmen oder beim Bestimmen davon zu helfen.

7. Robotisierte Vorrichtung (10) nach einem der Ansprüche 1 bis 6, wobei die Verarbeitungsschaltung (17) dazu konfiguriert ist, Parameter einer während des medizinischen Eingriffs durchzuführenden Behandlung durch Simulation der Auswirkungen der Parameter in Abhängigkeit von Bildern des Patienten anzupassen oder beim Anpassen davon zu helfen.

8. Robotisierte Vorrichtung (10) nach einem der Ansprüche 1 bis 7, die ein Führungswerkzeug (12) umfasst, das dazu ausgelegt ist, das medizinische Instrument (13) zu führen, das an einem Ende des Roboterarms (11) befestigt ist oder dazu bestimmt ist, daran befestigt zu sein.

9. Robotisierte Vorrichtung (10) nach Anspruch 8, wobei die Steuerschaltung (16) dazu konfiguriert ist, den Roboterarm (11) gemäß einem kollaborativen Verfolgungsmodus zu steuern, so dass, wenn ein Bediener Kräfte auf den Roboterarm (11) ausführt, um das Führungswerkzeug (12) im Verhältnis zum Positionssollwert und zum Ausrichtungssollwert abzulenken, der Roboterarm (11) Kräfte ausübt, die den vom Bediener ausgeführten Kräften entgegengesetzt sind, so dass das Führungswerkzeug (12) in den Positionssollwert und den Ausrichtungssollwert zurückgeführt wird, sobald keine Kräfte mehr vom Bediener ausgeübt werden.

10. Robotisierte Vorrichtung (10) nach einem der Ansprüche 8 bis 9, wobei das Führungswerkzeug (12) einen Messsensor für eine Einführungstiefe des medizinischen Instruments (13) umfasst.

11. Robotisierte Vorrichtung (10) nach einem der Ansprüche 1 bis 10, die mindestens ein Mensch-Maschine-Schnittstellengerät (19) aus den folgenden Geräten umfasst:
- einen Anzeigebildschirm,
- einen berührungsempfindlichen Bildschirm,
- eine Tastatur,
- 2D- und/oder 3D-Brillen,
- einen Steuerhebel,
- ein Bewegungsdetektionsmodul,
- ein sprachaktiviertes Steuermodul.

12. Robotisierte Vorrichtung (10) nach einem der Ansprüche 1 bis 11, die mindestens ein Eintrittspunkt-Ermittlungsgerät aus den folgenden Geräten umfasst:
- ein medizinisches Instrument mit einer atraumatischen Spitze,
- ein Laserzielmodul.

13. Robotisierte Vorrichtung (10) nach einem der Ansprüche 1 bis 12, wobei das medizinische Instrument (13) eines der folgenden medizinischen Instrumente ist:
- eine Biopsienadel,
- ein Katheter,
- ein Endoskop,
- ein Instrument zur Behandlung mit fokussiertem Ultraschall,
- ein Instrument zur Laserbehandlung,
- ein Instrument zur Behandlung durch Kryotherapie,
- ein Instrument zur Behandlung mit Radiofrequenz,
- ein Instrument zur Behandlung durch Elektroporation,
- ein Instrument zur Behandlung durch Brachytherapie.

14. Robotisierte Vorrichtung (10) nach einem der Ansprüche 1 bis 13, die einen mobilen Wagen (18) umfasst, der den Roboterarm (11) trägt, wobei der mobile Wagen Fixierungsmittel umfasst.

15. Robotisierte Vorrichtung (10) nach einem der Ansprüche 1 bis 14, wobei die Verarbeitungsschaltung (17) dazu konfiguriert ist, das biomechanische Modell zu verwenden, um lokale Deformationen der von dem medizinischen Instrument (13) durchquerten anatomischen Strukturen zu schätzen und um die Position eines Zielpunkts auf der Bewegungsbahn, dem das medizinischen Instrument (13) folgen soll, in Abhängigkeit von den geschätzten lokalen Deformationen zu aktualisieren.

## Claims

1. A robotic device (10) for carrying out a medical intervention on an anatomical area located inside the body of a patient (30) with a medical instrument (13), including:
- a robot arm (11) including several degrees of freedom, including one end adapted to receive the medical instrument,
- an image acquisition system (14) adapted to acquire position information relating to the anatomy of the patient,
- a storage medium (15) including a biomechanical model of the anatomical structures of the human body,
- a processing circuit (17) configured to transform the parameters of a trajectory to be followed by the medical instrument to carry out the medical intervention in the reference frame associated with the robotic device according to the biomechanical model and according to the position information of the anatomy of said patient, determine a position setpoint and an orientation setpoint for said medical instrument according to the trajectory,
- a control circuit (16) configured to control the robot arm (11) to place, or assist in placing, the medical instrument in the position setpoint and the orientation setpoint,
**characterized in that** the image acquisition system (14) is of the non-irradiating type and includes at least one so-called contactless equipment adapted to acquire position information without contact with the patient (30).

2. The robotic device (10) according to claim 1, wherein the biomechanical model models the anatomical structures of the human body in the thoracic area and/or the abdominal area and/or the pelvic area.

3. The robotic device (10) according to one of claims 1 to 2, wherein the image acquisition system (14) includes at least one amongst the following contactless equipment:
- a stereoscopic camera,
- a structured-light camera,
- a time-of-flight camera,
- a depth measurement camera.

4. The robotic device (10) according to one of claims 1 to 3, wherein the image acquisition system (14) is adapted to supply position information corresponding to the position of an outer surface of the body of the patient (30).

5. The robotic device (10) according to one of claims 1 to 4, wherein the control circuit (16) is configured to control the robot arm according to at least one amongst the following modes:
- an automatic mode,
- a collaborative mode,
- an automatic tracking mode,
- a collaborative tracking mode.

6. The robotic device (10) according to one of claims 1 to 5, wherein the processing circuit (17) is configured to determine or assist in determining the trajectory of the medical instrument according to images of the patient.

7. The robotic device (10) according to one of claims 1 to 6, wherein the processing circuit (17) is configured to set or assist in setting parameters of a treatment to be performed during the medical intervention by simulation of the effects of said parameters according to images of the patient.

8. The robotic device (10) according to one of claims 1 to 7, including a guide tool (12) adapted to guide the medical instrument (13), fastened or intended to be fastened at one end of the robot arm (11).

9. The robotic device (10) according to claim 8, wherein the control circuit (16) is configured to control the robot arm (11) according to a collaborative tracking mode so that, when an operator exerts efforts on the robot arm (11) to divert the guide tool (12) with respect to the position setpoint and the orientation setpoint, the robot arm (11) exerts efforts opposite to those exerted by the operator, so as to bring the guide tool (12) back in the position setpoint and the orientation setpoint as soon as no effort is exerted by said operator.

10. The robotic device (10) according to one of claims 8 to 9, wherein the guide tool (12) comprises a sensor for measuring a depth of insertion of the medical instrument (13) .

11. The robotic device (10) according to one of claims 1 to 10, including at least one human-machine interface equipment (19) from among the following equipment:
- a display screen,
- a display touchscreen,
- a keyboard,
- 2D and/or 3D goggles,
- a joystick,
- a movement detection module,
- a voice-control module.

12. The robotic device (10) according to one of claims 1 to 11, including at least one equipment for locating a point of entry from among the following equipment:
- a medical instrument with an atraumatic tip,
- a laser aiming module.

13. The robotic device (10) according to one of claims 1 to 12, wherein the medical instrument (13) is one amongst the following medical instruments:
- a biopsy needle,
- a catheter,
- an endoscope,
- an instrument for treatment by focused ultrasounds,
- a laser treatment instrument,
- an instrument for treatment by cryotherapy,
- an instrument for treatment by radiofrequency,
- an instrument for treatment by electroporation,
- an instrument for treatment by Curie-therapy.

14. The robotic device (10) according to one of claims 1 to 13, including a movable carriage (18) carrying the robot arm (11), said movable carriage including immobilizing means.

15. The robotic device (10) according to one of claims 1 to 14, wherein the processing circuit (17) is configured to use the biomechanical model to estimate local deformations of the anatomical structures crossed by the medical instrument (13) and to refresh the position of a target point of the trajectory that the medical instrument (13) should follow according to the estimated local deformations.
